# EUROPÄISCHE PATENTSCHRIFT

(11) **EP 0 725 052 B1**
(45) Veröffentlichungstag und Bekanntmachung des Hinweises auf die Patenterteilung: **06.05.1999**
(21) Anmeldenummer: 96100759.8
(22) Anmeldetag: 19.01.1996
(51) Int. Cl.: C07C 45/36, C07C 45/78, C07C 47/565

(54) **Verfahren zur Reinigung von 4-Hydroxybenzaldehyd enthaltenden Reaktionsgemischen**
Process for the purification of reaction mixtures containing 4-hydroxybenzaldehyde
Procédé pour la purification de mélanges de réaction contenant du 4-hydroxybenzaldéhyde

(30) Priorität: 01.02.1995 DE 19503163
(43) Veröffentlichungstag der Anmeldung: 07.08.1996
(73) Patentinhaber: BAYER AG, 51368 Leverkusen (DE)
(72) Erfinder: Schnatterer, Albert, Dr., D-51373 Leverkusen (DE); Fiege, Helmut, Dr., D-51373 Leverkusen (DE); Jelitto, Frank, Dr., D-51467 Bergisch Gladbach (DE); Skornia, Peter, D-53225 Bonn (DE); Theisen, Karl-Heinz, D-51061 Köln (DE)

(56) Entgegenhaltungen:
- EP-A- 0 209 798
- PATENT ABSTRACTS OF JAPAN vol. 014, no. 439 (C-0761), 19.September 1990 & JP-A-02 172942 (SUMITOMO CHEM CO LTD), 4.Juli 1990,

## Beschreibung

Die vorliegende Erfindung betrifft ein Verfahren zur Reinigung von 4-Hydroxybenzaldehyd enthaltenden Reaktionsgemischen wie sie bei der Direktoxidation von p-Kresol mit Sauerstoff anfallen.

4-Hydroxybenzaldehyd ist ein wichtiges Zwischenprodukt für die in Kosmetika als UV-Absorber eingesetzten Zimtsäureester. Darüber hinaus hat 4-Hydroxybenzaldehyd Bedeutung für die Synthese von Aromastoffen, Pharmazeutika und Pflanzenschutzprodukten.

Beim derzeit günstigsten Verfahren zur Herstellung von 4-Hydroxybenzaldehyd wird p-Kresol mit Sauerstoff oder einem Sauerstoff enthaltenden Gas in methanolischem Alkali und in Gegenwart von Metallverbindungen als Katalysatoren oxidiert. Geeignete Metallverbindungen sind beispielsweise Kobaltverbindungen (siehe EP-A 12 939), Eisen- und/oder Mangan-Chelatkomplexe (siehe EP-A 330 036) und mit Kobalt, Chrom oder Vanadin modifizierte mikroporöse Festkörper auf der Basis von Aluminium-/Silicium- und/oder Phosphoroxiden (siehe NL-A 92-968).

Nach der Oxidation liegt der 4-Hydroxybenzaldehyd in dem methanolisch-alkalischen Reaktionsgemisch gelöst als Alkalisalz vor. Das Reaktionsgemisch ist mit schwerlöslichen harzartigen Nebenprodukten verunreinigt. Diese Nebenprodukte beeinträchtigen die Qualität des isolierten 4-Hydroxybenzaldehyds und machen zusätzliche Reinigungsschritte erforderlich. Zusätzlich erschweren sie die Aufarbeitung des Reaktionsgemisches, weil sie die Emulsionsbildung zwischen Wasserphase und organischer Phase fördern, die Kristallisation von 4-Hydroxybenzaldehyd und seinen Salzen verzögern und zur Bildung sehr feiner und damit schwer filtrierbarer Kristalle des 4-Hydroxybenzaldehyds und seiner Salze führen. Außerdem kommt es auf Grund der harzartigen Konsistenz der Nebenprodukte zu Anbackungen und Verklebungen an Apparateteilen, insbesondere dann, wenn man 4-Hydroxybenzaldehyd nicht isolieren, sondern in Form des Reaktionsgemischs weiter umsetzen möchte, z.B. mit Methylchlorid zu Anisaldehyd.

Es ist also in jedem Fall günstig, diese harzartigen Nebenprodukte aus dem Reaktionsgemisch zu entfernen.

Für Reaktionsgemische aus der mit Kobaltverbindungen katalysierten Oxidation von p-Kresol ist es bekannt, den 4-Hydroxybenzaldehyd durch Kristallisation des Natriumsalzes aus wäßrigem Alkali, Abtrennung des Natriumsalzes und Freisetzung des 4-Hydroxybenzaldehyds nach erneutem Auflösen zu gewinnen (siehe JP-OS 02-172941 und JP-OS 63-216839).

Bei der Aufarbeitung über die Natriumsalz-Kristallisation erfolgt die Abtrennung der Nebenprodukte über die Mutterlauge. Die Nebenprodukte sind in wäßrigem Alkali gut löslich, in methanolischem Alkali jedoch nur wenig löslich.

Die Isolierung und Reinigung des 4-Hydroxybenzaldehyds über das Natriumsalz ist mit Ausbeuteverlusten durch Anteile in der Mutterlauge verbunden, apparativ aufwendig und somit kostenintensiv.

Es ist auch bekannt, 4-Hydroxybenzaldehyd durch Destillation zu isolieren (siehe JP-OS 01-106838). Diese Methode ist wegen den geringen thermischen Stabilität von rohem 4-Hydroxybenzaldehyd für eine Durchführung im technischen Maßstab nicht geeignet.

Die direkte Fitration der schwerlöslichen Nebenprodukte aus dem Reaktionsgemisch - eine Möglichkeit die an sich naheliegt - ist in Kombination mit einer nachgeschalteten Kristallisation des Natriumsalzes von 4-Hydroxybenzaldehyd beschrieben (siehe JP-OS 02-172942). Diese direkte Filtration ist unter betrieblichen Gegebenheiten jedoch ungünstig, da die abzutrennenden Nebenprodukte in schleimiger, sehr voluminöser Form mit hohen Anteilen an 4-Hydroxybenzaldehyd und Lösungsmitteln vorliegen. Die Filtration ist deshalb sehr langwierig (siehe vorliegendes Beispiel 5), die Wäsche der abfiltrierten schwerlöslichen Nebenprodukte wegen der schmierigen Konsistenz schwierig und mit erheblichem zusätzlichen Waschmittelbedarf verbunden.

Es wurde nun ein Verfahren zur Abtrennung von schwerlöslichen Nebenprodukten aus Reaktionsgemischen, die bei der Oxidation von p-Kresol mit Sauerstoff oder einem Sauerstoff enthaltenden Gas in Methanol als Lösungsmittel, in Gegenwart von Alkalihydroxid und in Gegenwart von Metallverbindungen als Katalysator anfallen, gefunden, das dadurch gekennzeichnet ist, daß man nach der Oxidation wenigstens 0,3 Mol Alkali pro Mol zur Oxidation eingesetztem Kresol mit Salzsäure, Schwefelsäure, Phosphorsäure, Salpetersäure, Kohlensäure oder Essigsäure neutralisiert, so daß ein Salzniederschlag entsteht, diesen Salzniederschlag zusammen mit den schwerlöslichen Nebenprodukten abtrennt und man bei der Neutralisation Wasser zufügt.

Die Neutralisation des überschüssigen Alkalis kann durch Zusatz von Säure erfolgen.

Bei der Oxidation von p-Kresol wird als Alkalihydroxid vorzugsweise Natriumhydroxid oder Kaliumhydroxid, insbesondere Natriumhydroxid eingesetzt. Bezogen auf p-Kresol kann man beispielsweise 2 bis 6 Mol-Äquivalente Alkalihydroxid einsetzen. Vorzugsweise beträgt diese Menge 2,5 bis 4,5 Mol-Äquivalente.

Das Methanol kann in reiner Form oder im Gemisch mit anderen Lösungsmitteln, z.B. mit anderen Alkoholen wie Ethylenglykol und/oder tert. Butanol und/oder im Gemisch mit Wasser eingesetzt werden. Vorzugsweise beträgt der Gehalt an anderen Lösungsmitteln weniger als 10 Gew.-%, bezogen auf Methanol.

Die Temperatur bei der Oxidation von p-Kresol kann z.B. zwischen 40 und 90°C liegen, bevorzugt sind Temperaturen zwischen 45 und 85°C.

Der Druck des Sauerstoffs bzw. des Sauerstoff enthaltenden Gases ist keiner besonderen Einschränkung unterworfen und kann z.B. zwischen 1 und 50 bar liegen. Vorzugsweise liegt er bei 1 bis 10 bar. Der Sauerstoffgehalt ist bei der Verwendung von Sauerstoff enthaltenden Gasen ebenfalls keiner Beschränkung unterworfen. Die wirtschaftlich günstigste Form ist der Einsatz von atmosphärischer Luft.

Als Katalysatoren können z.B. Verbindungen der Übergangsmetalle, z.B. Verbindungen des Vanadins, Chroms, Mangans, Eisens, Kobalts, Kupfers und/oder Cers eingesetzt werden. Bei den Verbindungen kann es sich z.B. um Oxide, Hydroxide und/oder Salze anorganischer Säuren handeln, beispielsweise um Fluoride, Chloride, Sulfate, Nitrate, Carbonate und/oder Phosphate und/oder um Salze organischer Säuren, beispielsweise um Acetate, Oxalate, Phenolate, Benzoate und/oder Salicylate und/oder um Komplexe dieser Metalle beispielsweise mitAcerylaceton, N,N'-Disalicylidenethylendiamin, Tetraarylporphinen und/oder Phthalocyaninen und/oder um mit diesen Metallen modifizierte Festkörper mit mikroporöser Struktur, z.B. so modifizierte Zeolithe. Bevorzugt eingesetzt werden Verbindungen des Kobalts, z.B. in Form der genannten Metallsalze und Metallkomplexe, Eisen- und/oder Mangan-Chelatkomplexe, insbesondere Eisen- und/oder Mangan-Tetraarylporphine und/oder mit Kobalt modifizierte Festkörper mit mikroporöser Struktur auf der Basis von Aluminium-, Silicium- und/oder Phosphoroxiden. Besonders bevorzugt sind Eisentetraarylporphine, gegebenenfalls in Kombination mit einer oder mehreren Kupferverbindungen als Co-Katalysatoren.

Einzelbeispiele für einsetzbare Eisentetraarylporphine sind Eisenkomplexe des Tetraphenylporphins, Tetrakis-(4-methoxyphenyl)-porphins, Tetrakis-(2-methoxyphenyl)-porphins, Tetrakis-(2-chlorphenyl)-porphins, Tetrakis-(2-hydroxyphenyl)-porphins und Tetrakis-(2,4-dimethoxyphenyl)-porphins.

Das molare Verhältnis von Katalysator, berechnet als Metall, zu eingesetztem p-Kresol kann z.B. 0,00001 bis 0,05 betragen. Vorzugsweise beträgt es 0,0001 bis 0,005.

Die Oxidation wird bevorzugt bis zum vollständigen Umsatz des p-Kresols durchgeführt. Man kann jedoch erfindungsgemäß auch Reaktionsgemische aufarbeiten, bei denen p-Kresol nur teilweise umgesetzt wurde, sowie Reaktionsgemische, welche die nicht vollständig oxidierten Zwischenstufen 4-Hydroxybenzylalkohol und/oder 4-Hydroxybenzylmethylether enthalten.

Die Säuren zur Erzeugung des Salzniederschlages können einzeln oder in beliebigen Gemischen eingesetzt werden. Bevorzugt wird Salzsäure oder Schwefelsäure verwendet.

Die Säuren können z.B. in reiner Form oder als wäßrige Lösungen eingesetzt werden. Der Wasseranteil solcher wäßrigen Lösungen kann in weitem Bereich variiert werden. Er ist lediglich der Einschränkung unterworfen, daß das eingeschleppte Wasser nicht die vollständige Lösung des Salzes bewirkt. Bevorzugt ist der Einsatz wasserfreier Säuren oder konzentrierter wäßriger Lösungen dieser Säuren, z.B. 10 bis 35 gew.-%ige Salzsäure und 70 bis 90 gew.-%ige Schwefelsäure. Die Temperatur des Reaktionsgemisches kann während der Säurezugabe z.B. zwischen 20°C und der Siedetemperatur des Reaktionsmediums variieren. Sie wird im allgemeinen so gewählt, daß gut abtrennbare Salzniederschläge anfallen. Im Falle des Natriumsulfats fällt vorzugsweise das grobkristalline Na₂SO₄ x 10 H₂O. Gute Ergebnisse werden im allgemeinen bei Temperaturen zwischen 20 und 75°C erzielt.

Der im Reaktionsgemisch nach der Oxidation vorliegende Alkaliüberschuß, der je nach Arbeitsweise z.B. 1 bis 6 Mol-Äquivalente, bevorzugt 1,5 bis 4 Mol-Äquivalente, betragen kann, kann erfindungsgemäß vollständig, d.h. bis zum Erreichen eines pH-Wertes von ca. 10, oder nur zum Teil neutralisiert werden. Man neutralisiert wenigstens 0,3 Mol Alkali pro Mol zur Oxidation eingesetztem p-Kresol. Wie weit die Neutralisation getrieben werden muß, um eine optimale Abtrennung der schwerlöslichen Nebenprodukte zusammen mit dem Salzniederschlag zu erreichen, hängt u.a. vom Wassergehalt im Reaktionsgemisch von der Art des Salzes und der Menge der Nebenprodukte ab und kann im Einzelfall durch einfache, routinemäßige Vorversuche leicht ermittelt werden.

Die Abtrennung des erfindungsgemäß gebildeten Niederschlags aus Salz und schwerlöslichen Nebenprodukten kann nach an sich bekannten Verfahren für die Feststoffabtrennung erfolgen, z.B. durch Filtration oder Sedimentation. Geeignete Apparaturen hierfür sind beispielsweise Drucknutschen, Bandfilter, Filterkerzen und Zentrifugen.

Überraschenderweise lassen sich durch die erfindungsgemäße Salzfällung die vorher schwer abtrennbaren und schwerlöslichen Nebenprodukte sehr gut von der Reaktionslösung abtrennen. Aufgrund des bekannten geringen Adsorptionsvermögens von anorganischen Salzen war es nicht vorherzusehen, daß die schwerlöslichen Nebenprodukte von dem Salzniederschlag praktisch vollständig gebunden und dadurch in eine ausgezeichnet abtrennbare Form gebracht werden können.

Nach der Abtrennung des Salzniederschlags zusammen mit den schwerlöslichen Nebenprodukten, läßt sich p-Hydroxybenzaldehyd harzfrei und in hoher Reinheit aus dem methanolischen Filtrat isolieren. Die vom Salzniederschlag und den schwerlöslichen Nebenprodukten befreiten Lösungen sind besonders geeignet zur direkten und damit kostengünstigen Weiterverarbeitung zu Folgeprodukten des 4-Hydroxybenzaldehyds. Eine kostenintensive Zwischenisolierung des 4-Hydroxy benzaldehyds oder seiner Alkalisalze zu Reinigungszwecken kann damit häufig entfallen. Probleme bei nachgeschalteten Aufarbeitungsoperationen, beispielsweise bei Phasentrennungen und Kristallisationen, treten nicht mehr auf. Beispiele für Folgeprodukte die sich durch direkte Weiterverarbeitung der vom Salzniederschlag und den schwerlöslichen Nebenprodukten befreiten Reaktionslösungen vorteilhaft herstellen lassen, sind Anisaldehyd, 4-Hydroxybenzonitril und der Aromastoff 1-(4-Hydroxyphenyl)-3-butanon.

### Beispiele

Prozentangaben beziehen sich auf das Gewicht, soweit nichts anderes gesagt ist.

### Beispiel 1

In einen 3 l-Glasreaktor mit Rührer, Rückflußkühler und Gaseinleitungsrohr wurden nacheinander 300 g p-Kresol (99 %ig), 0,10 g Eisen-tetrakis-(2,4-dimethoxyphenyl)-porphin-chlorid, 0,15 g Cu(NO₃)₂ x 3 H₂O, 1 500 g Methanol und 417 g Natriumhydroxid eingebracht. Das Einsatzgemisch wurde unter einer Stickstoffatmosphäre auf eine Temperatur von 55°C erwärmt und bei dieser Temperatur 90 Minuten gerührt. Es bildete sich eine homogene dunkelgrün gefärbte Lösung. In diese wurde anschließend unter kräftigem Rühren ein konstanter Strom von 40 l Luft/h eingeleitet. Der Sauerstoffgehalt im Abgas stellte sich anfangs auf ca. 6 Vol.-% ein. Nach 16 Stunden war die Umsetzung vollständig. Das Gewicht der Reaktionsmischung betrug dann 2 257 g und enthielt nach HPLC-Untersuchung kein p-Kresol, 13,7 % 4-Hydroxybenzaldehyd und 0,2 % 4-Hydroxybenzoesäure.

Danach wurden 220 ml wäßriger, 80 %iger Schwefelsäure bei einer Temperatur von 45 bis 50°C zugetropft. Danach lag der pH-Wert bei 11. Der Salzniederschlag enthaltend im wesentlichen Natriumsulfat und schwerlösliche Nebenprodukte wurde über eine Drucknutsche (Durchmesser 10 cm) bei 2,5 bar Überdruck abfiltriert. Dauer der Filtration: 18 Minuten. Das Filtrat war eine klare, rötlich gefärbte Lösung.

### Beispiel 2

Die Durchführung der Oxidation erfolgte wie in Beispiel 1. Nach Beendigung der Oxidation wurden dem Reaktionsgemisch 550 ml konz. wäßrige Salzsäure bei einer Temperatur von 60 bis 65°C zugetropft. Danach lag der pH-Wert bei 11. Der Niederschlag wurde wie in Beispiel 1 abfiltriert. Dauer der Filtration: 5 Minuten.

### Beispiel 3

Die Durchführung der Oxidation erfolgte wie in Beispiel 1. Nach Beendigung der Oxidation wurden 110 ml 80 %ige wäßrige Schwefelsäure zum Reaktionsgemisch getropft. Der Niederschlag wurde wie in Beispiel 1 abfiltriert. Dauer der Filtration: 15 Minuten.

### Beispiel 4

Die Durchführung der Oxidation erfolgte wie in Beispiel 1. Nach Beendigung der Oxidation wurden 55 ml 80 %ige wäßrige Schwefelsäure zum Reaktionsgemisch getropft. Der Niederschlag wurde wie in Beispiel 1 abfiltriert. Dauer der Filtration: 22 Minuten.

### Beispiel 5 (Vergleichsbeispiel - Filtration ohne vorhergehende Säurezugabe)

Die Durchführung der Oxidation erfolgte wie in Beispiel 1. Danach wurde das Reaktionsgemisch ohne vorherige Säurezugabe filtriert. Dauer der Filtration: 260 Minuten. Der Filtrationsrückstand bestand im wesentlichen aus schwerlöslichen Nebenprodukten.

### Beispiel 6 (Isolierung von 4-Hydroxybenzaldehyd)

1 000 g des Filtrates aus Beispiel 1 mit einem Gehalt von 13,9 % 4-Hydroxybenzaldehyd in Form des Natriumsalzes wurden einem destillativen Austausch von Methanol gegen Wasser unterzogen. Die zudosierte Wassermenge war so bemessen, daß das Volumen der wäßrigen Lösung nach vollständiger Methanoldestillation 770 ml betrug. Aus dieser Lösung wurde der 4-Hydroxybenzaldehyd bei 50°C durch Ansäuern mit 80 %iger wäßriger Schwefelsäure bis zu einem pH-Wert von 5,5 freigesetzt. Nach Abkühlen der Suspension auf 20°C wurde der 4-Hydroxybenzaldehyd abfiltriert, mit 150 ml Wasser nachgewaschen und getrocknet. Die Ausbeute betrug 135,4 g, der Gehalt war 99,1 %ig (gemäß HPLC).

### Beispiel 7 (Weiterverarbeitung des Filtrates aus Beispiel 1 zu Anisaldehyd)

1 000 g des Filtrates aus Beispiel 1 von einem Gehalt von 13,9 % 4-Hydroxybenzaldehyd in Form des Natriumsalzes wurden in einem 2 l-Edelstahlautoklaven bei 95°C und 6 bar mit 100 g Methylchlorid behandelt. Der pH-Wert im Reaktionsgemisch wurde über eine pH-Druckelektrode verfolgt und durch Nachdosieren von 45 %iger wäßriger Natronlauge zwischen 8,5 und 8,7 gehalten. Nach 8 Stunden Reaktionszeit war der Druck auf 2,5 bar gefallen. Das Reaktionsgemisch wurde auf Raumtemperatur abgekühlt, das Methanol unter Zusatz von 1 g Kaliumcarbonat abdestilliert und der Rückstand zur Lösung der Salze mit 350 ml Wasser verdünnt. Die nachfolgende Phasentrennung zwischen der wäßrigen Salzlösung und der Anisaldehydphase verlief problemlos. Die Anisaldehydphase wurde mit 50 ml Wasser gewaschen und anschließend bei 20 mbar destilliert. Hauptfraktion: 143,7 g mit einem Gehalt von 99,2 % Anisaldehyd.

## Patentansprüche

1. Verfahren zur Abtrennung von schwerlöslichen Nebenprodukten aus Reaktionsgemischen, die bei der Oxidation von p-Kresol mit Sauerstoff oder einem Sauerstoff enthaltenden Gas in Methanol als Lösungsmittel, in Gegenwart von Alkalihydroxid und von Metallverbindungen als Katalysator anfallen, dadurch gekennzeichnet, daß man nach der Oxidation mindestens 0,3 Mol Alkali pro Mol zur Oxidation eingesetztem p-Kresol mit Salzsäure, Schwefelsäure, Phosphorsäure, Salpetersäure, Kohlensäure oder Essigsäure neutralisiert, so daß ein Salzniederschlag entsteht, diesen Salzniederschlag zusammen mit schwerlöslichen Nebenprodukten abtrennt und man bei der Neutralisation Wasser zufügt.

2. Verfahren nach Anspruch 1, dadurch gekennzeichnet, daß man wäßrige, 10 bis 35 gew.-%ige Salzsäure oder wäßrige, 70 bis 90 gew.-%ige Schwefelsäure einsetzt.

3. Verfahren nach Ansprüchen 1 bis 2, dadurch gekennzeichnet, daß man bei der Oxidation als Alkalihydroxid Natriumhydroxid oder Kaliumhydroxid einsetzt.

4. Verfahren nach Ansprüchen 1 bis 3, dadurch gekennzeichnet, daß man bei der Oxidation als Metallverbindungen Verbindungen des Kobalts und/oder Eisen- und/oder Mangan-Chelatkomplexe und/oder mit Kobalt modifizierte mikroporöse Festkörper mit mikroporöser Struktur auf der Basis von Aluminium-, Silicium- und/oder Phosphoroxiden einsetzt.

5. Verfahren nach Ansprüchen 1 bis 4, dadurch gekennzeichnet, daß bei der Oxidation als Metallverbindungen Eisen- und/oder Mangantetraarylporphine eingesetzt werden.

6. Verfahren nach Ansprüchen 1 bis 5, dadurch gekennzeichnet, daß bei der Oxidation als Metallverbindungen Eisen-tetraarylphorphine, gegebenenfalls in Kombination mit einer oder mehreren Verbindungen des Kupfers zum Einsatz kommen.

## Claims

1. Process for separating off poorly soluble by-products from reaction mixtures which are produced in the oxidation of p-cresol with oxygen or an oxygen-containing gas in methanol as solvent in the presence of alkali metal hydroxide and of metal compounds as catalyst, characterized in that, after the oxidation, at least 0.3 mol of alkali per mole of p-cresol used for the oxidation is neutralized with hydrochloric acid, sulphuric acid, phosphoric acid, nitric acid, carbonic acid or acetic acid so that a salt precipitate forms, this salt precipitate is separated off together with poorly soluble by-products and water is added in the neutralization.

2. Process according to Claim 1, characterized in that aqueous 10 to 35% strength by weight hydrochloric acid or aqueous 70 to 90% strength by weight sulphuric acid is used.

3. Process according to Claims 1 to 2, characterized in that the alkali metal hydroxide used in the oxidation is sodium hydroxide or potassium hydroxide.

4. Process according to Claims 1 to 3, characterized in that the metal compounds used in the oxidation are compounds of cobalt and/or iron chelate complexes and/or manganese chelate complexes and/or alumina-, silica- and/or phosphorus oxide-based microporous solids of microporous structure modified by cobalt.

5. Process according to Claims 1 to 4, characterized in that the metal compounds used in the oxidation are iron tetraarylporphines and/or manganese tetraarylporphines.

6. Process according to Claims 1 to 5, characterized in that the metal compounds used in the oxidation are iron tetraarylporphines, if appropriate in combination with one or more compounds of copper.

## Revendications

1. Procédé pour séparer les produits d'accompagnement peu solubles de mélanges de réaction obtenus par oxydation du p-crésol à l'aide d'oxygène ou d'un gaz contenant de l'oxygène dans le méthanol servant de solvant, en présence d'un hydroxyde alcalin et de dérivés métalliques servant de catalyseurs, caractérisé en ce que, après l'oxydation, on neutralise au moins 0,3 mol d'alcali par mole du p-crésol mis en oeuvre à l'oxydation à l'aide d'acide chlorhydrique, d'acide sulfurique, d'acide phosphorique, d'acide nitrique, d'acide carbonique ou d'acide acétique, en formant ainsi un précipité de sel qu'on sépare avec les produits d'accompagnement peu solubles, la neutralisation étant accompagnée d'une addition d'eau.

2. Procédé selon la revendication 1, caractérisé en ce que l'on utilise un acide chlorhydrique aqueux à une concentration de 10 à 35 % en poids ou un acide sulfurique aqueux à une concentration de 70 à 90 % en poids.

3. Procédé selon les revendications 1 à 2, caractérisé en ce que, à l'oxydation, l'hydroxyde alcalin utilisé est l'hydroxyde de sodium ou l'hydroxyde de potassium.

4. Procédé selon les revendications 1 à 3, caractérisé en ce que, à l'oxydation, on utilise en tant que dérivés métalliques des dérivés du cobalt et/ou du fer et/ou des complexes du manganèse et/ou des corps solides microporeux à structure microporeuse à base d'alumine, de silice et/ou d'oxydes du phosphore, modifiés par du cobalt.

5. Procédé selon les revendications 1 à 4, caractérisé en ce que les dérivés métalliques utilisés à l'oxydation sont des complexes de fer et/ou de manganèse de tétraarylporphines.

6. Procédé selon les revendications 1 à 5, caractérisé en ce que les dérivés métalliques utilisés à l'oxydation sont des complexes de fer de tétraarylporphines, éventuellement en combinaison avec un ou plusieurs dérivés du cuivre.
